# FASCICULE DE BREVET EUROPEEN

(11) **EP 2 033 628 B1**
(45) Date de publication et mention de la délivrance du brevet: **24.10.2018**
(21) Numéro de dépôt: 08163551.8
(22) Date de dépôt: 03.09.2008
(51) Int. Cl.: A61K 8/99, A61Q 19/00, A61P 17/00, A61Q 5/00

(54) **Utilisation cosmétique de lysat des éspèces de Bifidobacterium pour le traitement de la sécheresse cutanée**
Kosmetischer Einsatz eines Lysats einer Bifidobakteriengattung zur Behandlung von Trockenheit der Haut
Cosmetic use of a Bifidobacterium species lysate for treating dry skin

(30) Priorité: 04.09.2007 FR 0757348
(43) Date de publication de la demande: 11.03.2009
(73) Titulaire: L'Oréal, 75008 Paris (FR)
(72) Inventeur: Castiel, Isabelle, 06200 NICE (FR); Breton, Lionel, 78000 VERSAILLES (FR); Gueniche, Audrey, 92500 RUEIL MALMAISON (FR)
(74) Mandataire: Nony

(56) Documents cités:
- EP-A- 0 043 128
- EP-A- 1 110 555
- EP-A- 1 344 528
- EP-A- 1 731 137
- WO-A1-2006/110631
- FR-A- 2 876 029
- FR-A1- 2 937 536

## Description

Le présent brevet vise principalement à proposer un nouvel actif pour la prévention et/ou le traitement de la sécheresse au niveau des matières kératiniques et notamment des peaux qualifiées de sèches.

Il est connu qu'une augmentation de la sécheresse cutanée est souvent observée avec l'âge, toutefois de tels états de sécheresse cutanée peuvent également se manifester chez les sujets jeunes. En effet, l'état de sécheresse cutanée est un état physiologique qui peut être présent chez des sujets jeunes, sans aucune cause pathologique, du moins apparente. L'origine de cette sécheresse peut être constitutionnelle ou acquise. Ainsi, de nombreux facteurs extérieurs peuvent entrainer l'assèchement de la peau ou aggraver l'état d'une peau déjà sèche. Parmi ces facteurs, on peut citer les conditions climatiques difficiles, les rayons solaires, l'exposition à certains agents chimiques ou thérapeutiques.

Sur le plan physiologique, la peau sèche est souvent associée à une baisse du taux d'hydratation cutanée et à une altération de la fonction barrière, mesurée par la perte insensible en eau. Sur le plan sensoriel, elle est notamment caractérisée par une sensation de tiraillement et/ou de tension cutanée. Pour des raisons évidentes, ces manifestations sont sources d'inconfort, voire de douleurs.

Il demeure donc un besoin de disposer de nouveaux actifs susceptibles d'exercer une action cosmétique ou thérapeutique bénéfique sur l'épiderme ou les matières kératiniques en général, qualifiées de sèches. Au sens de l'invention, on entend désigner par « épiderme » aussi bien la peau que le cuir chevelu.

EP 1 110 555 enseigne l'utilisation d'un microorganisme *Bifidobacterium lactis* pour le traitement de désordres tels que des états pelliculaires ; et WO 2006/110 631 décrit la mise en oeuvre par voie orale d'un microorganisme probiotique du genre *Bifidobacterium* pour traiter les états pelliculaires du cuir chevelu. Toutefois, ces microorganismes ne sont pas mis en oeuvre sous la forme d'un lysat.

De manière inattendue, les inventeurs ont constaté que certains microorganismes probiotiques peuvent s'avérer particulièrement efficaces pour la prévention et/ou le traitement des peaux sèches sous réserve qu'ils soient mis en oeuvre
sous la forme d'un lysat, ledit lysat étant formé de tous les constituants biologiques intracellulaires et des constituants des parois et membranes cellulaires.

En conséquence, selon un premier aspect, l'invention a pour objet l'utilisation cosmétique non-thérapeutique d'une quantité efficace d'un lysat d'au moins un microorganisme du genre *Bifidobacterium,* pour traiter et/ou prévenir une peau sèche choisie parmi une peau sèche constitutionnelle non
pathologique et une peau sèche acquise, ledit lysat étant formé de tous les constituants biologiques intracellulaires et des constituants des parois et membranes cellulaires.

Selon un autre de ses aspects, la présente invention vise un lysat d'au moins un microorganisme du genre *Bifidobacterium* en une quantité efficace, pour son utilisation pour traiter et/ou prévenir une peau sèche constitutionnelle pathologique, ledit lysat étant formé de tous les constituants biologiques intracellulaires et des constituants des parois et membranes cellulaires . En particulier, un tel lysat s'avère efficace pour traiter les ichtyoses, psoriasis, hyperkératoses, les dermatites topiques et les états pelliculaires secs du cuir chevelu.

La présente invention concerne selon un autre de ses aspects, l'utilisation cosmétique non-thérapeutique d'une quantité efficace d'un lysat d'au moins un microorganisme du genre *Bifidobacterium* pour traiter et/ou prévenir un état pelliculaire sec du cuir chevelu, et plus particulièrement les pellicules sèches, ledit lysat étant formé de tous les constituants biologiques intracellulaires et des constituants des parois et membranes cellulaires..

Selon un autre de ses aspects, la présente invention vise un lysat d'au moins un microorganisme du genre *Bifidobacterium* en une quantité efficace, pour son utilisation pour traiter et/ou prévenir un état pelliculaire sec du cuir chevelu, ledit lysat étant formé de tous les constituants biologiques intracellulaires et des constituants des parois et membranes cellulaires.

Au sens de la présente invention, le terme prévenir signifie diminuer le risque de manifestation du phénomène concerné.

Selon un autre de ses aspects, l'invention a pour objet un procédé cosmétique non-thérapeutique pour traiter et/ou prévenir une peau sèche choisie parmi une peau sèche constitutionnelle non pathologique et une peau sèche acquise
, chez un sujet comprenant au moins une étape d'administration audit sujet, d'au moins une quantité efficace d'un lysat d'au moins un microorganisme du genre *Bifidobacterium,* ledit lysat étant formé de tous les constituants biologiques intracellulaires et des constituants des parois et membranes cellulaires.

Selon un autre de ses aspects, la présente invention a pour objet une composition, cosmétique et/ou dermatologique, notamment utile pour prévenir et/ou traiter les matières kératiniques sèches, notamment les épidermes secs, tels que la peau ou le cuir chevelu, comprenant dans un support physiologiquement acceptable, au moins une quantité efficace d'un lysat d'au moins un microorganisme du genre *Bifidobacterium,* en association avec au moins une quantité efficace d'au moins un microorganisme notamment probiotique annexe, et/ou une de ses fractions et/ou un de ses métabolites, distinct dudit lysat, ledit lysat étant formé de tous les constituants biologiques intracellulaires et des constituants des parois et membranes cellulaires.

Au sens de l'invention, l'expression «distinct dudit lysat » signifie qu'il est possible de distinguer au sein de la composition soit deux microorganismes différents soit deux formes différentes d'un même microorganisme. Ainsi, lorsque le microorganisme annexe est du genre *Bifidobacterium* et correspond à la même espèce que celle figurant le lysat requis selon l'invention, ce microorganisme annexe est alors présent sous une forme autre qu'un lysat.

La présente description est aussi relative à une composition, cosmétique et/ou dermatologique, notamment utile pour prévenir et/ou traiter les matières kératiniques sèches, notamment les épidermes secs, tels que la peau ou le cuir chevelu, comprenant dans un support physiologiquement acceptable, au moins une quantité efficace d'un lysat d'au moins un microorganisme du genre *Bifidobacterium species*, et/ou une de ses fractions, en association avec une quantité efficace d'au moins un actif hydratant notamment tel que décrit ci-après.

Conviennent tout particulièrement à ce titre, l'urée et ses dérivés.

Selon une variante de réalisation de l'invention, le lysat selon l'invention peut être administré par voie orale.

Selon une autre variante de réalisation de l'invention, le lysat selon l'invention peut être administré par voie topique.

Comme précisé ci-après, les compositions le contenant sont formulées pour être compatibles avec le mode d'administration retenu.

Par « quantité efficace », on entend au sens de la présente invention une quantité suffisante pour obtenir l'effet attendu.

### Peau sèche

Comme signifié précédemment, la peau sèche se manifeste pour l'essentiel par une sensation de tiraillements et/ou de tension. Celle-ci est aussi rugueuse au toucher et apparaît couverte de squames. Lorsque la peau est légèrement sèche, ces squames sont abondantes mais peu visibles à l'oeil nu. Elles sont de moins en moins nombreuses mais de plus en plus visibles à l'oeil nu lorsque ce désordre s'aggrave.

Au niveau du cuir chevelu, la formation de tels squames ou pellicules sèches est symptomatique d'un état pelliculaire sec.

Au regard du cuir chevelu, les états pelliculaires secs sont des états chroniques, fréquents, récidivants, et socialement invalidants. Le stress et la période hivernale renforcent ces états chez la majorité des individus. L'intégrité et l'homéostasie du cuir chevelu sont régulées par un ensemble de paramètres parmi lesquels interviennent, la sécrétion de sébum et la sensibilité intra-individuelle.

Ainsi, lors des états pelliculaires du cuir chevelu, la barrière cutanée, son intégrité et son écoflore sont déséquilibrées.

La peau du cuir chevelu est irritée, et prurigineuse, fragile, moins hydratée et développe une desquamation importante se traduisant par un état pelliculaire sec.

Les états pelliculaires secs sont différents des états pelliculaires gras. Les premiers se distinguent notamment par la présence de squames grises ou blanches, de petites tailles et sèches, alors que les seconds se caractérisent par des squames larges, jaunes et grasses.

L'origine de cette sécheresse cutanée peut être de type constitutionnel ou acquis.

Dans le cas de la peau sèche constitutionnelle, on peut distinguer deux catégories : les peaux pathologiques et les peaux non pathologiques.

Les peaux sèches constitutionnelles pathologiques sont essentiellement représentées par la dermatite atopique et les ichthyoses. Elles sont quasiment indépendantes des conditions extérieures.

La dermatite atopique est décrite comme associée à un déficit dans le métabolisme des lipides du stratum corneum et notamment des céramides. Cette pathologie se présente sous la forme d'une xérose plus ou moins chronique concernant une grande étendue du corps, associée ou non à des poussées inflammatoires et prurigineuses par plaques.

Les dermatites atopiques sont également décrites comme des pathologies chroniques inflammatoires de la peau, coexistantes souvent avec d'autres pathologies atopiques tels que la rhinite, les conjonctivites, et l'asthme allergiques. Dans la plupart des cas la dermatite atopique se traduit par une peau sèche associée à des dysfonctionnements de la barrière épidermique. Une augmentation de la perte insensible en eau est presque toujours retrouvée.

Ainsi la fonction barrière de la peau est altérée non seulement sur les parties eczémateuses mais aussi au niveau de la peau sèche non inflammatoire. Cette altération facilite d'autant la pénétration de substances diverses de l'environnement dans la peau.

Qui plus est, une colonisation de la peau par des Staphylocoques doré (Staphylococcus aureus) est généralement corrélée à une dermatite atopique.

Les ichthyoses sont des pathologies caractérisées par un déficit génétique affectant le processus de kératinisation à différents stades. Elles se manifestent par une desquamation importante par plaques.

Les peaux sèches constitutionnelles pathologiques concernées selon l'invention sont plus particulièrement les peaux sèches ou cuirs chevelus secs d'origine non inflammatoire.

Dans le cas des peaux sèches constitutionnelles non pathologiques, la sévérité de l'état de sécheresse peut, pour sa part, dépendre de facteurs extérieurs. Rentrent dans cette catégorie de peau, la peau sénile (caractérisée par une diminution générale du métabolisme cutané avec l'âge), la peau fragile (très sensible aux facteurs extérieurs et souvent accompagnée d'érythème et de rosacée) et la xérose vulgaire (d'origine génétique probable et se manifestant en priorité sur le visage, les membres et le dos des mains).

Dans le cas de peau sèche acquise, l'intervention de paramètres extérieurs tels que l'exposition aux agents chimiques, à des conditions climatiques difficiles, aux rayons solaires ou bien encore certains traitements thérapeutiques (rétinoïdes, par exemple) est déterminante. Sous ces influences extérieures, l'épiderme peut devenir alors momentanément et localement sèche. Cela peut concerner tout type d'épiderme.

Ainsi, la sécheresse cutanée peut également être induite par un stress exogène, d'origine chimique, par exemple de type peeling, ou encore d'origine mécanique (frottements, rasage).

Il est rappelé qu'une opération de peeling consiste classiquement à appliquer sur la peau une substance chimique dans le but de provoquer une destruction limitée et contrôlée de l'épiderme et des couches superficielles du derme afin d'améliorer certains désordres de l'apparence cutanée.

Parallèlement aux peelings que l'on pourrait qualifier de chimiques au regard des produits chimiques qu'ils mettent en oeuvre, s'est également développée une technologie impliquant la mise en oeuvre de lasers ablatifs et non ablatifs.

Les premiers lasers ablatifs, réalisés avec des lasers CO₂ pulsés ou scannés, ont pour effet immédiat la vaporisation (ou ablation) de l'épiderme et souvent la partie supérieure du derme. Une bande du derme sous jacent est généralement également le siège d'une lésion thermique avec dénaturation et contraction du collagène. Pendant la phase de cicatrisation, il se produit une réepithélisation à partir des follicules pileux et d'autres annexes en plus d'une bande dermique supérieure (« remodelage du collagène »).

La dernière génération de lasers utilisent un système de transformation du faisceau laser en multitude de faisceaux espacés entre eux afin de produire sur la peau des impacts espacés entre eux, maintenant ainsi des zones de peau saine non altérée entre les zones touchées.

Pour des raisons évidentes, le peeling a donc une action qui, bien que contrôlée, demeure irritative au niveau de la surface de l'épiderme et de nature à induire une sécheresse cutanée.

Les compositions, procédés et utilisations selon la présente description, s'avèrent ainsi tout particulièrement efficaces :
- pour traiter les états de sécheresse cutanée, les états squameux et notamment les états pelliculaires secs,
- pour traiter les peaux sèches,
- pour traiter les démangeaisons et/ou tiraillements associés aux peaux sèches,
- pour traiter les désordres cutanés liés à un défaut d'excrétion et/ou de sécrétion de sébum,
- pour restaurer physiologiquement un état d'hydratation convenable au *stratum corneum*,
- pour traiter les peaux sèches hypo-séborrhéiques,
- pour stimuler la sébogénèse,
- pour prévenir et/ou réduire les rides liées à une sécheresse cutanée,
- pour améliorer le confort des peaux et cuirs chevelus secs, et notamment des états pelliculaires secs.
- pour lutter contre l'aspect terne et/ou atone de la peau conséquence de son dessèchement,
- pour traiter les fibres kératiniques sèches,

pour traiter les peaux ayant subies un stress exogène desséchant induit par un produit chimique tel qu'une composition de peeling par exemple, ou induit par un peeling par rayonnement ou encore induit mécaniquement notamment par frottement, au rasage par exemple.

Lorsque les matières kératiniques sont des fibres kératiniques humaines ou animales, à l'image des cheveux, poils et/ou cils, l'actif considéré s'avère particulièrement avantageux pour prévenir et/ou traiter la manifestation des signes de fragilité, comme par exemple la sécheresse qui se traduit, généralement, par un aspect cassant de la fibre. Il permet ainsi de conférer un aspect lustré aux fibres kératiniques, en particulier à la chevelure humaine et au pelage animal.

Selon un mode de réalisation de l'invention un lysat de microorganismes conforme à l'invention n'est pas mis en oeuvre à titre d'agent antiadhésion de la flore pathogène de la peau.

### Microorganismes

Comme précisé précédemment, les microorganismes du genre *Bifidobacterium species* utilisés à titre d'actifs selon l'invention sont mis en oeuvre sous la forme d'un lysat.

Un lysat désigne communément un matériau obtenu à l'issue de la destruction ou dissolution de cellules biologiques par un phénomène dit de lyse cellulaire provoquant ainsi la libération des constituants biologiques intracellulaires naturellement contenus dans les cellules du microorganisme considéré.

Au sens de la présente description, le terme lysat est utilisé indifféremment pour désigner l'intégralité du lysat obtenu par lyse du microorganisme concerné ou seulement une fraction de celui-ci.

Le lysat mis en oeuvre est formé de tous les constituants biologiques intracellulaires et des constituants des parois et membranes cellulaires.

Plus précisément, il contient la fraction cytoplasmique cellulaire renfermant les enzymes tels que la déhydrogénase d'acide lactique, les phosphatases, les phosphokétolases, et transaldolases. A titre illustratif, les constituants des parois cellulaires
sont notamment le peptidoglycane, la muréine ou mucopeptide et l'acide teichoique et les constituants des membranes cellulaires sont composés de glycérophospholipide.

Cette lyse cellulaire peut être accomplie par différentes technologies, telles que par exemple choc osmotique, choc thermique, par ultrasons, ou encore sous contrainte mécanique de type centrifugation par exemple.

Plus particulièrement, ce lysat peut être obtenu selon la technologie décrite dans le brevet US 4 464 362 et notamment selon le protocole suivant.

Le microorganisme de type *Bifidobacterium* considéré est cultivé anaerobiquement dans un milieu de culture adéquat, par exemple selon les conditions décrites dans les documents US 4 464 362 et EP 43 128. Lorsque la phase stationnaire du développement est atteinte, le milieu de culture peut être inactivé par pasteurisation, par exemple à une température de 60 à 65 °C pendant 30 mn. Les microorganismes sont alors recueillis par une technique de séparation conventionnelle par exemple filtration membranaire, centrifugation et remis en suspension dans une solution NaCl physiologique stérile.

Le lysat peut être obtenu par désintégration aux ultras-sons d'un tel milieu afin d'en libérer les fractions cytoplasmiques, les fragments de paroi cellulaire et les produits issus du métabolisme. Puis tous les composants dans leur distribution naturelle sont ensuite stabilisés dans une solution aqueuse faiblement acide.

On obtient ainsi généralement une concentration de l'ordre de 0,1 à 50 %, en particulier de 1 à 20 % et notamment environ 5 % en poids en matière(s) active(s) par rapport au poids total du lysat.

Le lysat peut être mis en oeuvre sous différentes formes, sous la forme d'une solution ou sous une forme pulvérulente.

Le microorganisme appartenant au genre *Bifidobacterium* est plus particulièrement choisi parmi les espèces : *Bifidobacterium longum Bifidobacterium bifidum, Bifidobacterium breve, Bifidobacterium animalis, Bifidobacterium lactis, Bifidobacterium infantis, Bifidobacterium adolescentis* ou *Bifidobacterium pseudocatenulatum* et leurs mélanges.

Convient tout particulièrement à l'invention, l'espèce *Bifidobacterium longum.*

Au sens de l'invention, le terme fraction désigne plus particulièrement un fragment dudit microorganisme doté d'une efficacité pour le traitement des épidermes secs par analogie audit microorganisme entier.

Le produit commercialisé sous la dénomination Repair Complex CLR® par la société K. RICHTER GmbH et qui est formé d'un lysat inactivé de l'espèce *Bifidobacterium longum*, entre dans le cadre de l'invention.

L'actif formant le lysat appartenant au genre Bifidobacterium peut être formulé à raison d'au moins 0,0001 % (exprimé en poids sec), en particulier à raison de 0,001 à 20% et plus particulièrement à raison de 0,001 à 2 % en poids par rapport au poids total du support ou de la composition le contenant.

Dans le cas particulier où le(s) microorganisme(s) est (sont) formulé(s) dans des compositions à administrer par voie orale, la concentration en microorganisme(s) notamment probiotique(s) peut être ajustée de manière à correspondre à des doses (exprimées en équivalent de microorganisme) variant de 5.10² à 10¹³ ufc/j et en particulier de 10⁵ à 10¹¹ ufc/j.

Selon une variante de l'invention, ce lysat est mis en oeuvre en association avec un autre microorganisme.

Ainsi, les compositions selon l'invention peuvent avantageusement en outre contenir au moins un microorganisme annexe, notamment de type probiotique et/ou l'une de ses fractions et/ou l'un de ses métabolites.

Au sens de la présente invention, on entend par « microorganisme probiotique», un microorganisme vivant qui, lorsqu'il est consommé en quantité adéquate, a un effet positif sur la santé de son hôte «joint FAO/WHO Expert Consultation on Evaluation of Health and Nutritional Properties of Probiotic in Food Including Powder Milk with Live Lactic Acid Bacteria, 6 octobre 2001 », et qui peut en particulier améliorer l'équilibre microbien intestinal.

Ces microorganismes convenant à l'invention peuvent être choisis notamment parmi les ascomycetes telles que *Saccharomyces*, *Yarrowia*, *Kluyveromyces*, *Torulaspora*, *Schizosaccharomyces pombe*, *Debaromyces*, *Candida*, *Pichia*, *Aspergillus* et *Pénicillium*, des bactéries du genre *Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus et Lactobacillus* et leurs mélanges.

Comme ascomycetes convenant tout particulièrement à la présente invention, on peut en particulier citer *Yarrowia lipolitica et Kluyveromyces lactis,* de même que *Saccharomyces cereviseae, Torulaspora, Schizosaccharamyces pombe, Candida* et *Pichia.*

Des exemples spécifiques de microorganismes probiotiques sont *Lactobacillus acidophiltis, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus paracasei, Lactobacillus rhamnosus (Lactobacillus GG), Lactobacillus sake, Lactococcus lactis, Streptococcus thermophilus, Staphylococccus carnosus,* et *Staphylococcus xylosus et leurs mélanges.*

Plus particulièrement, il s'agit de microorganismes probiotiques issus du groupe des bactéries lactiques, comme notamment les *Lactobacillus.* A titre illustratif de ces bactéries lactiques, on peut plus particulièrement citer les *Lactabacillus johnsonii, Lactobacillus paracasei, Lactobacillus reuteri, Lactobacillus rhamnosus* et leurs mélanges.

Au sens de l'invention, le terme métabolite désigne toute substance issue du métabolisme des microorganismes considérés selon l'invention et dotée également d'une efficacité pour le traitement des épidermes secs.

Comme précisé précédemment, le microorganisme annexe peut être ou non de même espèce que celui formant le lysat. Toutefois, lorsqu'il est de même espèce, il est alors présent sous une forme autre qu'un lysat, par exemple sous une forme vivante.

Les espèces convenant tout particulièrement sont les *Lactobacillus johnsonii,* notamment la souche déposée suivant le traité de Budapest avec l'Institut Pasteur (28 rue du Docteur Roux, F-75024 Paris cedex 15) sous la désignation suivante CNCM I-1225.

D'une manière générale, les compositions selon l'invention comprennent généralement de 0,0001 à 20 %, en particulier de 0,001 à 15 % et plus particulièrement de 0,1 à 10 % en un ou plusieurs microorganismes notamment probiotiques, annexes.

Ce ou ces microorganisme(s) peu(ven)t être inclus dans les compositions selon l'invention sous une forme vivante, semi-active ou inactivée, morte.

Il(s) peu(ven)t également être inclus sous forme de fractions de composants cellulaires.

Le ou le(s) microorganisme(s), métabolite(s) ou fraction(s) peu(ven)t également être introduit(s) sous la forme d'une poudre, d'un liquide, d'un surnageant de culture ou l'une de ses fractions, dilué(e) ou non, ou encore concentré(e) ou non.

Dans le cas où les microorganismes sont formulés dans une composition sous une forme vivante, la quantité de microorganismes vivants peut varier de 10³ à 10¹⁵ ufc/g, en particulier de 10⁵ à 10¹⁵ ufc/g et plus particulièrement de 10⁷ à 10¹² ufc/g de microorganismes par gramme de composition.

Les compositions selon l'invention peuvent se présenter sous toutes les formes galéniques normalement disponibles pour le mode d'administration retenu.

Le support peut être de nature diverse selon le type de composition considérée.

En ce qui concerne plus particulièrement les compositions destinées à une administration par voie topique externe c'est-à-dire en surface d'une matière kératinique telle que la peau, il peut s'agir de solutions aqueuses, hydroalcooliques ou huileuses, de dispersions du type des solutions ou dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions, du type crème, de gel aqueux ou anhydre, de microémulsions, de microcapsules, de microparticules, ou de dispersions vésiculaires de type ionique et/ou non ionique.

Ces compositions sont préparées selon les méthodes usuelles.

Ces compositions peuvent notamment constituer des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après-rasage, des crèmes épilatoires, ou des compositions contre les piqûres d'insectes.

Les compositions selon l'invention peuvent également consister en des préparations solides constituant des savons ou des pains de nettoyage.

Elles peuvent être également utilisées pour le cuir chevelu sous forme de solutions, de crèmes, de gels, d'émulsions, de mousses ou encore sous forme de compositions pour aérosol contenant également un agent propulseur sous pression.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique et/ou dermatologique. L'émulsionnant et le coémulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, les formes galéniques dédiées à une administration topique peuvent contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, les acides gras insaturés et les huiles fluorées comme par exemple les perfluoropolyéthers. On peut aussi utiliser des alcools gras, des acides gras comme par exemple l'acide stéarique et comme par exemple des cires notamment de paraffine, carnauba et la cire d'abeilles. On peut aussi utiliser des composés siliconés comme les huiles siliconées et par exemple les cyclométhicone et diméthicone, les cires, les résines et les gommes siliconées.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO® par la société HENKEL, le mélange de PEG-6/PEG-32/Glycol Stéarate vendu sous la dénomination de Tefose® 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20OE).

Comme solvants utilisables dans l'invention, on peut citer les alcools inférieurs, notamment l'éthanol et l'isopropanol, le propylène glycol.

La composition de l'invention peut également contenir de façon avantageuse une eau thermale et/ou minérale, notamment choisie parmi l'eau de Vittel, les eaux du bassin de Vichy et l'eau de la Roche Posay.

Comme gélifiants hydrophiles, on peut citer les polymères carboxyliques tel que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305® par la société SEPPIC, les polysaccharides comme les dérivés cellulosiques tels que les hydroxyalkylcelluloses et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Dans le cas d'une utilisation d'une association conforme à l'invention par voie orale, on privilégie l'utilisation d'un support ingérable.

Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suppléments oraux sous forme sèche et les suppléments oraux sous forme liquide.

Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, capsules. En particulier, le(s) actif(s) selon l'invention peuvent être incorporés dans toute autre forme de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Selon un mode de réalisation particulier, les microorganismes annexes considérés selon l'invention peuvent être formulés au sein de compositions sous une forme encapsulée de manière à améliorer significativement leur durée de survie. Dans un tel cas, la présence d'une capsule peut en particulier retarder ou éviter la dégradation du microorganisme au niveau du tractus gastro intestinal.

Bien entendu, les compositions topiques ou orales, ou associations selon l'invention peuvent en outre contenir plusieurs autres actifs.

A titre d'actifs conventionnellement mis en oeuvre, on peut citer, les vitamines B3, B5, B6, B8, C, E, ou PP, la niacine, les caroténoïdes, les polyphénols et minéraux tels que zinc, calcium, magnésium ....

En particulier, on peut utiliser un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, l'hespéridine, des proanthocyanidines et des anthocyanines.

Il peut également s'agir d'au moins un prébiotique ou un mélange de prébiotiques. Plus particulièrement, ces prébiotiques peuvent être choisis parmi les oligosaccharides, produits à partir du glucose, galactose, xylose, maltose, sucrose, lactose, amidon, xylane, l'hémicellulose, l'inuline, des gommes de type acacia par exemple, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline.

Dans les formes galéniques topiques, on peut utiliser plus particulièrement comme actifs hydrophiles les protéines ou les hydrolysats de protéine, les acides aminés, les polyols notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Pour ce qui est des actifs lipophiles, on peut utiliser le rétinol (vitamine A) et ses dérivés, le tocophérol (vitamine E) et ses dérivés, les céramides, les huiles essentielles et les insaponifiables (tocotriènol, sésamine, gamma oryzanol, phytostérols, squalènes, cires, terpènes).

Selon un mode de réalisation une composition de l'invention est dépourvue de vitamine A.

Selon une variante de l'invention, le lysat conforme à l'invention peut être mis en oeuvre dans une composition topique avec un agent actif à l'égard des épidermes notamment secs.

A titre illustratif et non limitatif de tels actifs, on peut en particulier citer les actifs hydratants.

Par « actif hydratant », on entend :
- soit un composé agissant sur la fonction barrière, en vue de maintenir l'hydratation du stratum *corneum,* ou un composé occlusif. On peut citer les céramides, les composés à base sphingoides, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques, la vaseline, et la lanoline;
- soit un composé augmentant directement la teneur en eau du stratum *corneum,* tel que l'urée et ses dérivés, le thréalose et ses dérivés, l'acide hyaluronique et ses dérivés, le glycérol, le pentanediol, les pidolates, la sérine, le xylitol, l'acide lactique et le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine.
- Soit un composé activant les glandes sébacées tels que les dérivés stéroidiens (dont la DHEA) et la vitamine D et ses dérivés.

Ces composés peuvent représenter de 0,001% à 30%, et de préférence de 0,01 à 20%, du poids total de la composition selon l'invention.

A titre illustratif des dérivés d'urée, on peut plus particulièrement citer les dérivés hydroxyalkyl urées et notamment ceux décrits dans le document FR 2 877 222.

Comme actifs susceptibles d'être plus particulièrement associés au lysat dans une formule galénique orale, on peut également considérer tous les ingrédients communément utilisés et/ou autorisés.

A titre illustratif, on peut citer les vitamines, les minéraux, les lipides essentiels, les oligoéléments, les polyphénols, les flavonoïdes, les phytoestrogènes, les antioxydants tels que l'acide lipoïque et le coenzyme Q10, les caroténoïdes, les prébiotiques, les protéines et les acides aminés, les mono et polysaccharides, les amino-sucres, les phytostérols et alcools triterpéniques d'origine végétale.

Il s'agit, en particulier, des vitamines A, C, D, E, PP et du groupe B. Parmi les caroténoïdes, on choisit de préférence, le béta-carotène, le lycopène, la lutéine, la zéazanthine et l'astaxanthine. Les minéraux et oligo-éléments particulièrement mis en oeuvre sont le zinc, le calcium, le magnésium, le cuivre, le fer, l'iode, le manganèse, le sélénium, le chrome (III). Parmi les polyphénols, on retient aussi en particulier les polyphénols de raisin, de thé, d'olive, de cacao, de café, de pomme, de myrtille, de sureau, de fraise, de canneberge, et d'oignon. De préférence parmi les phytoestrogènes, on retient les isoflavones sous la forme libre ou glycosylée, telles que la génistéine, la daidzéine, la glycitéine ou encore les lignanes, en particulier ceux du lin et du schizandra chinensis. Les acides aminés ou les peptides et les protéines les contenant, tels que la taurine, la thréonine, la cystéine, le tryptophane, la méthionine. Les lipides appartiennent de préférence au groupe des huiles contenant des acides gras mono et polyinsaturés tels que les acides oléique, linoléique, alpha-linolénique, gamma-linolénique, stearidonique, les acides gras oméga-3 de poisson à longue chaîne tels que l'EPA et le DHA, les acides gras conjugués issus de végétaux ou d'animaux tels que les CLA (Conjugated Linoleic Acid).

Le procédé de traitement cosmétique non-thérapeutique de l'invention peut être mis en oeuvre notamment en administrant les compositions cosmétiques et/ou
dermatologiques ou associations telles que définies ci-dessus, selon la technique d'utilisation habituelle de ces compositions. Par exemple : applications de crèmes, de gels, de sérums, de lotions, de laits de démaquillage ou de compositions après-solaires sur la matière kératinique telle que la peau ou les cheveux secs, application d'une lotion pour cheveux sur cheveux mouillés ou de shampooings pour ce qui est de l'application topique.

Le procédé cosmétique non-thérapeutique selon l'invention peut être ainsi mis en oeuvre par administration topique, journalière par exemple, du lysat considéré selon l'invention.

Le procédé selon l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les ingrédients sont mélangés, avant leur mise en forme, dans l'ordre et dans des conditions facilement déterminées par l'homme de l'art.

Les exemples ci-après sont présentés à titre illustratif et non limitatif du domaine de l'invention.

| **Exemple 1 :** Lait pour le soin du visage des peaux sèches | % en poids |
|---|---|
| Chlorure de magnésium | 3,00 |
| Ascorbate de calcium | 3,00 |
| *Bifidobacterium longum* lysat CLR (Repair Complex CLR®)* | 10,00** |
| Stéarate de glycérol | 1,00 |
| Alcool cétylstéarylique/alcool cétylstéarylique oxyéthyléné à 30 moles OE (Sinnowax AO® vendu par la société HENKEL) | 3,00 |
| Alcool cétylique | 1,00 |
| Diméthicone (DC 200 Fluid® vendu par la société DOW CORNING) | 1,00 |
| Huile de vaseline | 6,00 |
| Myristate d'isopropyle (Estol IMP 1514 vendu® par UNICHEMA) | 3,00 |
| Antioxydant | 0,05 |
| Glycérine | 20,00 |
| Conservateur | 0,30 |
| Eau | qsp 100 |

| | |
|---|---|
| * Repair Complex CLR® commercialisé par K.Richter GmbH et correspondant à une formulation à 5 % en poids d'actifs ** quantité exprimée en produit total | |

### Exemple 2 :

| Lait pour le soin du visage des peaux sèches | % en poids |
|---|---|
| Ascorbate de magnésium | 3,00 |
| Huile de pépin de cassis | 4,00 |
| Huile de bourrache | 4,00 |
| Poudre de *Lactobacillus johnsonii* sous forme inactivée | 5,00 |
| *Bifidobacterium longum* lysat CLR (Repair Complex CLR®)* | 10,00** |
| Stéarate de glycérol | 1,00 |
| Alcool cétylstéarylique/alcool cétylstéarylique oxyéthyléné à 3 moles OE (Sinnovax AO® vendu par la société HENKEL) | 3,00 |
| Alcool cétylique | 1,00 |
| Diméthicone (DC 200 Fluid® vendu par la société Dow Corning) | 1,00 |
| Huile de vaseline | 6,00 |
| Myristate d'isopropyle (Estol IPM 1514® vendu par la société Unichema) | 3,00 |
| Glycérine | 20,00 |
| Conservateur | 0,30 |
| Eau | qsp 100 |

| | |
|---|---|
| * Repair Complex CLR® commercialisé par K.Richter GmbH et correspondant à une formulation à 5 % en poids d'actifs ** quantité exprimée en produit total | |

### Exemple 3

| La lotion pour le cuir chevelu | % en poids |
|---|---|
| *Bifidobacterium longum* lysat CLR (Repair Complex CLR®)* | 5,00** |
| Antioxydant | 0,05 |
| Isopropanol | 40,0 |
| Conservateur | 0,30 |
| Eau | qsp 100 |

| | |
|---|---|
| * Repair Complex CLR® commercialisé par K.Richter GmbH et correspondant à une formulation à 5 % en poids d'actifs ** quantité exprimée en produit total | |

### Exemple 4

| Lait pour le soin du cuir chevelu | % en poids |
|---|---|
| *Bifidobacterium longum* lysat CLR (Repair Complex CLR®)* | 5,00** |
| Stéarate de glycérol | 1,00 |
| Alcool cétylstéarylique/alcool cétylstéarylique oxyéthyléné à 30 moles OE (Sinnowax AO® vendu par la société HENKEL) | 3,00 |
| Alcool cétylique | 1,00 |
| Diméthicone (DC 200 Fluid® vendu par la société DOW CORNING) | 1,00 |
| Huile de vaseline | 6,00 |
| Myristate d'isopropyle (Estol IMP 1514 vendu® par UNICHEMA) | 3,00 |
| Antioxydant | 0,05 |
| Glycérine | 20,00 |
| Conservateur | 0,30 |
| Eau | qsp 100 |

| | |
|---|---|
| * Repair Complex CLR® commercialisé par K.Richter GmbH et correspondant à une formulation à 5 % en poids d'actifs ** quantité exprimée en produit total | |

### Exemple 5

| Gel pour le soin du cuir chevelu | % en poids |
|---|---|
| *Bifidobacterium longum* lysat CLR (Repair Complex CLR®)* | 5,00** |
| Hydroxypropylcellulose (Klucel H® vendu par la société | 5,00 |
| HERCULES) | 1,00 |
| Vitamine E | 2,50 |
| Antioxydant | 0,05 |
| Isopropanol | 40,00 |
| Conservateur | 0,30 |
| Eau | qsp 100 |

| | |
|---|---|
| * Repair Complex CLR® commercialisé par K.Richter GmbH et correspondant à une formulation à 5 % en poids d'actifs ** quantité exprimée en produit total | |

### Exemple 6

| Crème pour le soin du cuir chevelu | % en poids |
|---|---|
| Arachidyl behenyl alcohol/arachidylglusoside | 3,0 |
| Isohexadecane | 7,0 |
| *Bifidobacterium longum* lysat CLR (Repair Complex CLR®)* | 5,00** |
| Glycérine | 2,0 |
| Extrait de *Vitreoscillafiliformis* | 3,0 |
| BHT | 0,05 |
| POB méthyle | 0,1 |
| POB propyle | 0,05 |
| Eau | qsp 100 |

| | |
|---|---|
| * Repair Complex CLR® commercialisé par K.Richter GmbH et correspondant à une formulation à 5 % en poids d'actifs ** quantité exprimée en produit total | |

### Exemple 7

| Gel pour le soin des cheveux | % en poids |
|---|---|
| *Bifidobacterium longum* lysat CLR (Repair Complex CLR®)* | 5,00** |
| Citrate de Cuivre | 2,00 |
| Extrait de *Vitreoscilla filiformis* | 3,00 |
| Antioxydant | 0,05 |
| Vitamine C | 2,50 |
| Antioxydant | 0,05 |
| Isopropanol | 40,00 |
| Conservateur | 0,30 |
| Eau | qsp100,00 |

| | |
|---|---|
| * Repair Complex CLR® commercialisé par K.Richter GmbH et correspondant à une formulation à 5 % en poids d'actifs ** quantité exprimée en produit total | |

### Exemple 8

| Lotion pour le visage | % en poids |
|---|---|
| Lysat de *Bifidobacterium longum* (Repair Complex CLR®)* | 5,00** |
| Antiinflammatoire | 0,05 |
| Antioxydant | 0,05 |
| Isopropanol | 40,0 |
| Conservateur | 0,30 |
| Eau | qsp 100% |

| | |
|---|---|
| * Repair Complex CLR® commercialisé par K.Richter GmbH et correspondant à une formulation à 5 % en poids d'actifs ** quantité exprimée en produit total | |

### Exemple 9

| Gel pour le soin du visage | % en poids |
|---|---|
| Lysat de *bifidobacterium longum* (Repair Complex CLR®)* | 5,00 |
| Hydroxypropylcellulose (Klucel H® vendu par la société HERCULES) | 5,00 |
| | 1,00 |
| Vitamine E | 2,50 |
| Antioxydant | 0,05 |
| Isopropanol | 40,00 |
| Conservateur | 0,30 |
| Eau | qsp 100 |

| | |
|---|---|
| * Repair Complex CLR® commercialisé par K.Richter GmbH et correspondant à une formulation à 5 % en poids d'actifs ** quantité exprimée en produit total | |

### Exemple 10

| Crème pour le soin du visage | % en poids |
|---|---|
| Arachidyl behenyl alcohol/arachidylglusoside | 3,0% |
| Isohexadecane | 7,0% |
| Lysat de *Biofidobacterium longum* (Repair Complex CLR®)* | 5,00 |
| Glycérine | 2,0% |
| Extrait de Vitreoscilla filiformis | 3,0% |
| BHT | 0,05% |
| POB méthyle | 0,1% |
| POB propyle | 0,05% |
| Eau | qsp 100% |

| | |
|---|---|
| * Repair Complex CLR® commercialisé par K.Richter GmbH et correspondant à une formulation à 5 % en poids d'actifs ** quantité exprimée en produit total | |

### Exemple 11

| Gel pour le soin du visage | % en poids |
|---|---|
| Extrait de Vitreoscilla filiformis | 3,00 |
| Lysat de *Bifidobacterium longum* (Repair Complex CLR) | 5 |
| Antioxydant | 0,05 |
| Vitamine C | 2,50 |
| Antioxydant | 0,05 |
| Isopropanol | 40,00 |
| Conservateur | 0,30 |
| Eau | qsp 100,00 |

| | |
|---|---|
| * Repair Complex CLR® commercialisé par K.Richter GmbH et correspondant à une formulation à 5 % en poids d'actifs ** quantité exprimée en produit total | |

### Exemple 12

Evaluation de la sécheresse de sujets traités par un lysat de bifidobacterium.

Le produit testé est un lysat de *Bifidobacterium longum* en suspension désintégrée (aux ultrasons) dans un milieu aqueux faiblement acide commercialisé sous le nom Repair Complex CLR®.

L'actif a été testé seul dans une étude randomisée en double aveugle.

Soixante six femmes présentant des peaux sèches ont été réparties en deux groupes, placebo (n=33 groupe A), Repair Complex CLR® (n=33 groupe B). Les traitements ont été appliqués topiquement durant 58 jours, l'actif étant formulé à 10 % de la formulation testée. Cette formule support est une émulsion H/E déminéralisée Arlacel/myrj® contenant 5 % Parleam, 15 % de cyclopentasiloxane, 3% glycérine et 2 % vaseline.

Dans la formule placebo, l'absence de Repair Complex CLR® est compensée par de l'eau.

Les sujets ont été évalués à J1, J29, J43, et J57. A chaque visite, des évaluations de la sécheresse des jambes ont été réalisées par le dermatologue et par autoévaluation par les sujets selon les modalités précisées ci-après.

Le dermatologue investigateur a évalué à chaque visite la sécheresse cutanée de la zone étudiée (au niveau de la face externe de la jambe droite) selon une échelle de 0 à 3 sur les critères suivants : 0 = peau non sèche, 1 = sécheresse légère (rugosité légère), 2 = sécheresse modérée (rugosité modérée, quelques squames), 3 = sécheresse sévère (rugosité et desquamation importantes).

De plus, le dermatologue investigateur a demandé à chaque visite au sujet une auto-évaluation de l'état de sécheresse cutanée au niveau de ses jambes selon l'échelle de 0 à 5 suivante : 0= pas du tout ; 1= très légèrement ; 2= légèrement ; 3= moyennement ; 4= fortement ; 5= très fortement.

Parallèlement une étude de l'évolution de différents marqueurs cutanés par proteomique a été effectuée.

Un prélèvement est effectué au niveau de la face externe de la jambe aux temps J1, J29, J43 et J57 par stripping vernis afin de ne prélever qu'une partie du *stratum corneum,* soit au maximum 4 à 5 couches de *stratum corneum.*

Une toile de nylon filtre 41 µm type NY41 Millipore (5 x 5 cm) est appliquée sur une zone préalablement définie de la jambe gauche. Puis un vernis transparent de référence 614254/T.D. comprenant : nitrocellulose 6,86g ; isopropanol 2,94g ; résine alkyle hypoallergénique 7,35g ; acetyl tributyl citrate 7,7g ; acétate d'éthyle 75,15g ; est étalé à l'aide d'un pinceau (15mm) puis laissée sécher pendant 15 min. La toile de nylon est ensuite récupérée à l'aide d'une pince Brucelles en arrachant d'un coup sec le stripping vernis.

Les strippings vernis sont conservés à -20°C à plat dans des sachets plastiques.

Ces prélèvements de peau (stripping vernis de stratum corneum) ont ensuite été analysés par protéomique pour évaluer l'expression de différentes protéines selon la méthode décrite par Zieske (J. Exp. Bot., 2006, 547 : 1501) et Wiese et al., (Proteomics, 2007, 7, 340).

### Résultats

### a) Par scorage clinique

Le tableau 1 ci-après représente, par visite et traitement, les modalités du score de sécheresse cutanée exprimées en pourcentage. Les groupes sont comparables à J1 (p=0.8677).

On observe une amélioration au cours du temps dans les deux groupes qui est plus marquée pour le groupe étant traité par la formule topique contenant 10% de Repair Complex CLR® et tout particulièrement à J29.

**Tableau 1**

| **clinique** | | | | | | | | |
|---|---|---|---|---|---|---|---|---|
| | | | | jambes | | | | Total |
| | | | | 0 | 1 | 2 | 3 | |
| A | Temps | J1 | Effectif | 0 | 0 | 21 | 11 | 32 |
| | | | % dans Temps | ,0% | ,0% | 65,6% | 34,4% | 100,0% |
| | | J15 | Effectif | 7 | 8 | 15 | 2 | 32 |
| | | | % dans Temps | 21,9% | 25,0% | 46,9% | 6,3% | 100,0% |
| | | J29 | Effectif | 4 | 18 | 7 | 1 | 30 |
| | | | % dans Temps | 13,3% | 60,0% | 23,3% | 3,3% | 100,0% |
| | | J43 | Effectif | 8 | 15 | 3 | 3 | 29 |
| | | | % dans Temps | 27,6% | 51,7% | 10,3% | 10,3% | 100,0% |
| | | J57 | Effectif | 13 | 10 | 4 | 1 | 28 |
| | | | % dans Temps | 46,4% | 35,7% | 14,3% | 3,6% | 100,0% |
| | Total | | Effectif | 32 | 51 | 50 | 18 | 151 |
| | | | % dans Temps | 21,2% | 33,8% | 33,1% | 11,9% | 100,0% |
| B | Temps | J1 | Effectif | 0 | 0 | 21 | 10 | 31 |
| | | | % dans Temps | ,0% | ,0% | 67,7% | 32,3% | 100,0% |
| | | J15 | Effectif | 5 | 16 | 9 | 0 | 30 |
| | | | % dans Temps | 16,7% | 53,3% | 30,0% | ,0% | 100,0% |
| | | J29 | Effectif | 8 | 19 | 3 | 0 | 30 |
| | | | % dans Temps | 26,7% | 63,3% | 10,0% | ,0% | 100,0% |
| | | J43 | Effectif | 3 | 24 | 2 | 1 | 30 |
| | | | % dans Temps | 10,0% | 80,0% | 6,7% | 3,3% | 100,0% |
| | | J57 | Effectif | 11 | 15 | 4 | 0 | 30 |
| | | | % dans Temps | 36,7% | 50,0% | 13,3% | ,0% | 100,0% |
| | Total | | Effectif | 27 | 74 | 39 | 11 | 151 |
| | | | % dans Temps | 17,9% | 49,0% | 25,8% | 7,3% | 100,0% |

La diminution du score clinique de sécheresse au cours du temps apparaît très significative (Chi-Square test, p<0.0001). Ainsi, on observe une différence significative entre les groupes à J29 (Chi-square test en unilatérale ; p=0,0612) en faveur du traitement par la formule topique contenant 10 % de Repair Complex CLR®.

### b) Par auto-évaluation

Le tableau 2 ci-dessous représente, par visite et traitement, les modalités du score de sécheresse cutanée auto évaluée, exprimée en pourcentage. Les groupes sont comparables à J1 (p=0.3945).

On observe une amélioration au cours du temps dans les deux groupes qui s'avère plus marquée pour le groupe traité par la formule topique contenant 10 % de Repair Complex CLR®, particulièrement à J29, de façon comparable à ce qui avait été observé pour le score clinique.

**Tableau 2**

| | | | | jambes | | | | | | Total |
|---|---|---|---|---|---|---|---|---|---|---|
| rando | | | | 0 | 1 | 2 | 3 | 4 | 5 | |
| A | Temps | J1 | Effectif | 0 | 0 | 2 | 9 | 15 | 6 | 32 |
| | | | % dans Temps | ,0% | ,0% | 6,3% | 28,1% | 46,9% | 18,8% | 100,0% |
| | | J15 | Effectif | 2 | 11 | 6 | 11 | 2 | 0 | 32 |
| | | | % dans Temps | 6,3% | 34,4% | 18,8% | 34,4% | 6,3% | ,0% | 100,0% |
| | | J29 | Effectif | 4 | 6 | 9 | 11 | 0 | 0 | 30 |
| | | | % dans Temps | 13,3% | 20,0% | 30,0% | 36,7% | ,0% | ,0% | 100,0% |
| | | J43 | Effectif | 5 | 9 | 9 | 5 | 1 | 0 | 29 |
| | | | % dans Temps | 17,2% | 31,0% | 31,0% | 17,2% | 3,4% | ,0% | 100,0% |
| | | J57 | Effectif | 6 | 7 | 9 | 5 | 1 | 0 | 28 |
| | | | % dans Temps | 21,4% | 25,0% | 32,1% | 17,9% | 3,6% | ,0% | 100,0% |
| | Total | | Effectif | 17 | 33 | 35 | 41 | 19 | 6 | 151 |
| | | | % dans Temps | 11,3% | 21,9% | 23,2% | 27,2% | 12,6% | 4,0% | 100,0% |
| B | Temps | J1 | Effectif | 0 | 0 | 5 | 10 | 9 | 7 | 31 |
| | | | % dans Temps | ,0% | ,0% | 16,1% | 32,3% | 29,0% | 22,6% | 100,0% |
| | | J15 | Effectif | 6 | 5 | 10 | 6 | 3 | 0 | 30 |
| | | | % dans Temps | 20,0% | 16,7% | 33,3% | 20,0% | 10,0% | ,0% | 100,0% |
| | | J29 | Effectif | 8 | 9 | 7 | 5 | 1 | 0 | 30 |
| | | | % dans Temps | 26,7% | 30,0% | 23,3% | 16,7% | 3,3% | ,0% | 100,0% |
| | | J43 | Effectif | 6 | 13 | 8 | 3 | 0 | 0 | 30 |
| | | | % dans Temps | 20,0% | 43,3% | 26,7% | 10,0% | ,0% | ,0% | 100,0% |
| | | J57 | Effectif | 8 | 14 | 5 | 3 | 0 | 0 | 30 |
| | | | % dans Temps | 26,7% | 46,7% | 16,7% | 10,0% | ,0% | ,0% | 100,0% |
| | Total | | Effectif | 28 | 41 | 35 | 27 | 13 | 7 | 151 |
| | | | % dans Temps | 18,5% | 27,2% | 23,2% | 17,9% | 8,6% | 4,6% | 100,0% |

On a comparé les groupes aux différentes visites. On observe une différence significative entre les groupes à J29 (Chi-square test en unilatérale ; p=0,0561) en faveur du groupe traité avec la formule topique contenant 10% de Repair Complex CLR®.

### c) Analyse par protéomique

Les résultats de l'analyse par protéomique montrent que le lysat de *Bifidobactérium longum* stimule l'expression de différentes protéines de défenses antimicrobiennes de l'épiderme telles que la Ribonucléase 7, la dermicidine, la prolactine-inducible protein (PIP), les protéines S100 A8 et A9, et la protéine histone, de certaines protéases impliquées dans le phénomène de desquamation (KLK7, KLK5, Cathepsin L2) et de certaines protéines impliquées dans la maturation de coméodesmosome alors que d'autres protéines, témoignant de l'immaturité métabolique de la barrière cutanée, voient leur expression diminuer (Bleomycin hydrolase, Enolase 1, TPI, GAPDH).

Les propriétés de défense de la peau contre la sécheresse sont ainsi renforcées.

### Conclusion

Pour la sécheresse des jambes (aussi bien par la clinique, que les autoévaluations), on observe une diminution significative à J29 et globalement après 2 mois une tendance à la diminution pour les sujets qui ont été traités par la formule topique contenant 10 % de Repair Complex CLR®.

## Revendications

1. Utilisation cosmétique non-thérapeutique d'une quantité efficace d'un lysat d'au moins un microorganisme du genre *Bifidobacterium* pour traiter et/ou prévenir une peau sèche choisie parmi une peau sèche constitutionnelle non pathologique et une peau sèche acquise, ledit lysat étant formé de tous les constituants biologiques intracellulaires et des constituants des parois et membranes cellulaires.

2. Utilisation cosmétique non-thérapeutique d'une quantité efficace d'un lysat d'au moins un microorganisme du genre *Bifidobacterium* pour traiter et/ou prévenir un état pelliculaire sec du cuir chevelu, ledit lysat étant formé de tous les constituants biologiques intracellulaires et des constituants des parois et membranes cellulaires.

3. Lysat d'au moins un microorganisme du genre *Bifidobacterium* en une quantité efficace, pour son utilisation pour traiter et/ou prévenir une peau sèche constitutionnelle pathologique, ledit lysat étant formé de tous les constituants biologiques intracellulaires et des constituants des parois et membranes cellulaires.

4. Utilisation selon l'une quelconque des revendications 1 à 2, dans laquelle le microorganisme du genre *Bifidobacterium* est le *Bifidobacterium longum.*

5. Utilisation selon l'une quelconque des revendications 1 à 2 et 4, dans laquelle ledit lysat comprend de 0,1 à 50 % en poids, en particulier de 1 à 20 % en poids de matière(s) active(s) dérivée(s) du microorganisme du genre *Bifidobacterium.*

6. Composition cosmétique et/ou dermatologique, notamment utile pour prévenir et/ou traiter les matières kératiniques sèches, comprenant dans un milieu physiologiquement acceptable, au moins une quantité efficace d'un lysat d'au moins un microorganisme du genre *Bifidobacterium* en association à au moins une quantité efficace d'au moins un microorganisme annexe, et/ou une de ses fractions et/ou un de ses métabolites, distinct dudit lysat, ledit lysat étant formé de tous les constituants biologiques intracellulaires et des constituants des parois et membranes cellulaires.

7. Composition selon la revendication 6, dans laquelle le lysat est tel que défini en revendications 4 ou 5.

8. Composition selon la revendication 6 ou 7, dans laquelle ledit lysat est présent à raison d'au moins 0,0001 % (exprimé en poids sec), en particulier à raison de 0,001 à 20% et plus particulièrement à raison de 0,001 à 2 % en poids par rapport au poids total de ladite composition.

9. Composition selon l'une des revendications 6 à 8, dans laquelle ledit microorganisme annexe est choisi parmi les ascomycètes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus* et leurs mélanges.

10. Composition selon l'une quelconque des revendications 6 à 9, dans laquelle le microorganisme annexe et/ou une de ses fractions et/ou un de ses métabolites est présent à raison de 0,0001 à 20 %, en particulier de 0,001 à 15 % et notamment de 0,1 à 10 % en poids de ladite composition.

11. Composition selon l'une quelconque des revendications 6 à 10, **caractérisée en ce qu'**elle se présente sous forme de solutions aqueuses, hydroalcooliques ou huileuses, de dispersions du type des solutions ou dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème, de gel aqueux ou anhydre, ou encore de microémulsions, de microcapsules, de microparticules, ou de dispersions vésiculaires de type ionique et/ou non ionique.

12. Procédé cosmétique non-thérapeutique pour traiter et/ou prévenir une peau sèche choisie parmi une peau sèche constitutionnelle non pathologique et une peau sèche aquise chez un sujet comprenant au moins une étape d'administration audit sujet, d'au moins une quantité efficace d'un lysat d'au moins un microorganisme du genre *Bifidobacterium,* ledit lysat étant formé de tous les constituants biologiques intracellulaires et des constituants des parois et membranes cellulaires.

13. Procédé selon la revendication 12, dans lequel ledit microorganisme est tel que défini selon l'une quelconque des revendications 4 ou 5.

## Patentansprüche

1. Kosmetische nichttherapeutische Verwendung einer wirksamen Menge eines Lysats von mindestens einem Mikroorgsanismus der Gattung Bifidobacterium zur Behandlung und/oder Verhinderung trockener Haut, ausgewählt aus konstitutioneller nicht pathologischer Haut und erworbener trockener Haut, wobei das Lysat aus sämtlichen biologischen intrazellulären Bestandteilen und Zellwand- und Zellmembran-Bestandteilen gebildet ist.

2. Kosmetische nicht therapeutische Verwendung einer wirksamen Menge eines Lysat von mindestens einem Mikroorgsanismus der Gattung Bifidobacterium zur Behandlung und/oder Verhinderung eines Hauttrockenheitszustands der Kopfhaut, wobei das Lysat aus sämtlichen biologischen intrazellulären Bestandteilen und Zellwand- und Zellmembran-Bestandteilen gebildet ist.

3. Lysat von mindestens einem Mikroorgsanismus der Gattung Bifidobacterium in einer wirksamen Menge zu seiner Verwendung zur Behandlung und/oder Verhinderung konstitutioneller pathologischer Haut, wobei das Lysat aus sämtlichen biologischen intrazellulären Bestandteilen und Zellwand- und Zellmembran-Bestandteilen gebildet ist.

4. Verwendung nach einem der Ansprüche 1 bis 2, wobei der Mikroorgsanismus der Gattung Bifidobacterium Bifidobacterium longum ist.

5. Verwendung nach einem der Ansprüche 1 bis 2 und 4, wobei das Lysat von 0,1 bis 50 Gew.-%, insbesondere von 1 bis 20 Gew.-% 20 Wirkstoff(e), die sich von dem Mikroorgsanismus der Gattung Bifidobacterium ableiten, umfasst.

6. Kosmetische und/oder dermatologische Zusammensetzung, die insbesondere zur Verhinderung und/oder Behandlung trockener keratinischer Substanzen geeignet ist, umfassend, in einem physiologisch verträglichen Medium, mindestens einer wirksamen Menge eines Lysats von mindestens einem Mikroorgsanismus der Gattung Bifidobacterium in Kombination mit mindestens einer wirksamen Menge von mindestens einem Begleitmikroorgsanismus und/oder einer seiner Fraktionen und/oder einem seiner Metaboliten, die von dem Lysat verschieden sind, wobei das Lysat aus sämtlichen biologischen intrazellulären Bestandteilen und Zellwand- und Zellmembran-Bestandteilen gebildet ist.

7. Zusammensetzung nach Anspruch 6, wobei das Lysat wie in den Ansprüchen 4 oder 5 definiert ist.

8. Zusammensetzung nach Anspruch 6 oder 7, wobei das Lysat zu mindestens 0,0001 % (ausgedrückt in Trockengewicht), insbesondere zu 0,001 bis 20% und spezieller zu 0,001 bis 2 Gew.-% bezogen auf das Gessamtgewicht der Zusammensetzung vorhanden ist.

9. Zusammensetzung nach einem der Ansprüche 6 bis 8, wobei der Begleitmikroorgsanismus ausgewählt ist aus Ascomyzeten, wie Saccharomyces, Yarrowia, Kluyveromyces, Torulaspora, Schizosaccharomyces pombe, Debaromyces, Candida, Pichia, Aspergillus und Penicillium, Bakterien der Gattung Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus und ihren Gemischen.

10. Zusammensetzung nach einem der Ansprüche 6 bis 9, wobei der Begleitmikroorgsanismus und/oder eine seiner Fraktionen und/oder einer seiner Metaboliten zu 0,0001 bis 20 %, insbesondere zu 0,001 bis 15 % und vor allem 0,1 bis 10 Gew.-% der Zusammensetzung vorhanden ist.

11. Zusammensetzung nach einem der Ansprüche 6 bis 10,
**dadurch gekennzeichnet**, das sie in Form von wässrigen, wässrig-alkoholischen oder öligen Lösungen, Dispersionen vom Typ der Lösungen oder Dispersionen vom Typ einer Lotion oder eines Serums, Emulsionen flüssiger oder halbflüssiger Konsistenz vom Typ einer Milch, erhalten durch Dispersion einer Fettphase in einer wässrigen Phase (H/E) oder umgekehrt (E/H), oder Suspensionen oder Emulsionen weicher, halbfester oder fester Konsistenz von Typ einer Creme, wässrigem oder wasserfreiem Gel, oder auch Mikroemulsionen, Mikrokapseln, Mikroteilchen, oder Vesikeldispersionen vom ionischen und/oder nicht ionischen Typ dargereicht wird.

12. Kosmetisches nicht therapeutisches Verfahren zur Behandlung und/oder Verhinderung trockener Haut, ausgewählt aus nicht pathologischer konstitutioneller trockener Haut und erworbener trockener Haut bei einem Individuum, umfassend mindestens einen Schritt der Verabreichung an das Individuum von mindestens einer wirksamen Menge eines Lysats von mindestens einem Mikroorgsanismus der Gattung Bifidobacterium, wobei das Lysat aus sämtlichen biologischen intrazellulären Bestandteilen und Zellwand- und Zellmembran-Bestandteilen gebildet ist.

13. Verfahren nach Anspruch 12, wobei der Mikroorgsanismus wie nach einem der Ansprüche 4 oder 5 definiert ist.

## Claims

1. Non-therapeutic cosmetic use of an effective amount of a lysate of at least one microorganism of the genus *Bifidobacterium* for treating and/or preventing dry skin selected from a non-pathological constitutional dry skin and an acquired dry skin, wherein the lysate is formed of all intracellular biological constituents and constituents of cell walls and membranes.

2. Non-therapeutic cosmetic use of an effective amount of a lysate of at least one microorganism of the genus *Bifidobacterium* for treating and/or preventing a dry dandruff condition of the scalp, wherein the lysate is formed of all intracellular biological constituents and constituents of cell walls and membranes.

3. Lysate of at least one microorganism of the genus *Bifidobacterium* in an effective amount, for use in treating and/or preventing pathological constitutional dry skin, wherein the lysate is formed of all intracellular biological constituents and constituents of cell walls and membranes.

4. Use according to any one of the claims 1 to 2, wherein the microorganism of the genus *Bifidobacterium* is *Bifidobacterium longum.*

5. Use according to any one of the claims 1 to 2 and 4, wherein the lysate comprises from 0.1 to 50% by weight, in particular from 1 to 20% by weight of active material (s) derived from the microorganism of the genus *Bifidobacterium.*

6. Cosmetic and/or dermatological composition, especially useful for preventing and/or treating dry keratin materials, comprising in a physiologically acceptable medium, at least one effective amount of a lysate of at least one microorganism of the genus *Bifidobacterium* in combination with at least one effective amount of at least one additional microorganism and/or a fraction thereof and/or one of its metabolites, distinct from the lysate, wherein the lysate is formed of all intracellular biological constituents and constituents of cell walls and membranes.

7. Composition according to claim 6, wherein the lysate is as defined in claims 4 or 5.

8. Composition according to claim 6 or 7, wherein the lysate is present in an amount of at least 0.0001% (expressed in dry weight), in particular of 0.001 to 20% and more particularly of 0.001 to 2% by weight relative to the total weight of the composition.

9. Composition according to one of the claims 6 to 8, wherein the aditional microorganism is selected from ascomycetes such as *Saccharomyces, Yarrowia, Kluyveromyces, Torulaspora, Schizosaccharomyces pombe, Debaromyces, Candida, Pichia, Aspergillus and Penicillium;* bacteria of the genus *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus,* and mixtures thereof.

10. Composition according to any one of the claims 6 to 9, wherein the additional microorganism and/or one of its fractions and/or one of its metabolites is present in a proportion of 0.0001 to 20%, in particular of 0.001 to 15%, and especially of 0.1 to 10% by weight of the composition.

11. Composition according to any one of the claims 6 to 10, **characterized in that** it is in the form of aqueous, hydroalcoholic or oily solutions, of dispersions in the form of solutions or dispersions of the lotion or serum type, of emulsions with liquid or semi-liquid consistency of the milk type, obtained by dispersion of a fatty phase in an aqueous phase (O/W) or conversely (W/O), or suspensions or emulsions of soft semi-solid or solid consistency of the cream type, of cream, of aqueous or anhydrous gel, or of microemulsions, of microcapsules, of microparticles, or of ionic and/or nonionic type vesicular dispersions.

12. Non-therapeutic cosmetic method for treating and/or preventing dry skin selected from a non-pathological constitutional dry skin and an acquired dry skin in a subject comprising at least one step of administering to the subject at least one effective amount of a lysate of at least one microorganism of the genus *Bifidobacterium,* wherein the lysate is formed of all the intracellular biological constituents and constituents of the cell walls and membranes.

13. Method according to claim 12, wherein the microorganism is as defined in any one of the claims 4 or 5.
